# EUROPEAN PATENT APPLICATION

(11) **EP 4 663 120 A1**
(43) Date of publication of application: **17.12.2025**
(21) Application number: 25179607.4
(22) Date of filing: 28.05.2025
(51) Int. Cl.: A61B 5/145, A61B 5/1486, A61B 5/00, G16H 40/63

(54) **METHOD OF DISPLAYING COMMUNICATION STATUS IN GLUCOSE MONITORING SYSTEM**

(30) Priority: 12.06.2024 KR 20240076488
(71) Applicant: i-Sens, Inc., Seoul 06646 (KR)
(72) Inventor: KIM, Kyu Jin, Incheon (KR)
(74) Representative: HGF

(57) **Abstract**

One embodiment may provide a method of displaying a communication status in a glucose monitoring system, the method including displaying a communication status of a communication module in a terminal configured to transmit and receive data with a sensor transmitter, when the communication module is turned off, delaying a display of a communication module turn-off for one period of time by the terminal, and if the turn-off of the communication module persists for the one period of time, displaying the communication module turn-off.

## Description

### CROSS-REFERENCE TO RELATED PATENT APPLICATION

The present application claims priority under 35 U.S.C. § 119(a) to Korean patent application number 10-2024-0076488 filed on June 12, 2024, in the Korean Intellectual Property Office, the entire disclosure of which is incorporated by reference herein.

### BACKGROUND OF THE INVENTION

### 1. Field

Embodiments of the present disclosure relate to a method of displaying a communication status in a glucose monitoring system, and more specifically, to a technology for displaying a communication status to notify when a communication module is turned off.

### 2. Description of the Related Art

Recently, with the advancement of medical technology, various medical devices that are attached to a user's body have been developed and sold. A medical device attached to the user's body may be useful for monitoring biometric information or providing treatment by being attached to the skin of a chronic disease patient.

For example, chronic diseases such as diabetes require continuous management, and a medical device that is attached to the skin and measures glucose may be used to monitor glucose in diabetic patients. Diabetes is characterized by almost no noticeable symptoms in the early stages, but as the disease progresses, symptoms specific to diabetes appear, such as polydipsia, polyphagia, polyuria, weight loss, general malaise, itchy skin, and wounds on the hands and feet that do not heal and persist for a long time. As diabetes progresses further, complications such as vision impairment, high blood pressure, kidney disease, stroke, periodontal disease, muscle spasms, neuralgia, and gangrene appear. In order to diagnose diabetes and manage it to prevent it from developing into complications, systematic glucose measurement and treatment must be carried out together.

For diabetic patients and people who have not developed diabetes but have more sugar than normal detected in their blood, many medical device manufacturers provide various types of glucose meters that may measure glucose.

There are two types of glucose meters: one that measures blood glucose levels on a one-time basis by drawing blood from the user's fingertip, and one that measures blood glucose levels continuously by attaching the meter to the user's stomach or arm.

In the case of diabetic patients, they generally go back and forth between hyperglycemia and hypoglycemia, and emergency situations occur during hypoglycemia, and loss of consciousness or prolonged hypoglycemia without sugar supply may result in death. Therefore, immediate detection of hypoglycemia is very important for diabetic patients, but glucose meters that measure glucose intermittently have limitations in accurately detecting this condition.

Recently, to overcome these limitations, a continuous glucose monitoring system (CGMS), which is inserted into the human body and measures glucose levels at intervals of several minutes, has been developed and used. In order to minimize the user's pain and resistance following blood collection, the CGMS may measure glucose continuously after inserting a needle-shaped transdermal sensor into areas where pain is relatively less, such as the stomach or arm.

The CGMS includes a sensor transmitter that is inserted into the user's skin to measure glucose in the body and transmit the measured glucose level, and a terminal that outputs the received glucose level.

The terminal may be connected to the sensor transmitter and communicate with each other. In addition, the terminal may visually output this communication status to the user. Since the terminal must provide the user with a glucose level in real time and continuously receive biometric information for the glucose level from the sensor transmitter, it is essential to secure a stable communication status. Therefore, the terminal divides the communication status into several cases and displays it in various ways to inform the user of the communication status and allows the user to respond immediately when a communication problem occurs. Recently, the communication status is visually provided to the user through an application provided by the terminal, and in particular, it is necessary to accurately determine when the communication module is turned off and to promptly inform the user of an off state of the communication module through the application.

### SUMMARY OF THE INVENTION

Against this background, one object of embodiments of the present disclosure is to accurately determine the off state in which the communication module is turned off and to display the communication status in a timely manner.

Against this background, another object of embodiments of the present disclosure is to display the communication status after a predetermined period of time during which the display is delayed in order to determine the turn-off of the communication module.

In order to achieve the above described objects, an embodiment may provide a method of displaying a communication status in a glucose monitoring system, the method including displaying a communication status of a communication module in a terminal configured to transmit and receive data with a sensor transmitter; when the communication module is turned off, delaying a display of a communication module turn-off for one period of time by the terminal; and if the turn-off of the communication module persists for the one period of time, displaying the communication module turn-off.

In the method, the communication status may indicate a Bluetooth communication connection between the terminal and the sensor transmitter.

In the method, the one period of time may be preset or input by a user.

In the method, the method may further include performing determination on whether to display the communication module turn-off, wherein the displaying of the communication module turn-off may include displaying the communication module turn-off based on a result of the determination.

In the method, the performing of the determination on whether to display the communication module turn-off may include determining whether to display the communication module turn-off based on whether a communication failure persists in the terminal for the one period of time, and the displaying of the communication module turn-off may include, if the communication failure persists for the one period of time, displaying the communication module turn-off.

In the method, the communication failure may include a case where a communication connection between the terminal and the sensor transmitter is disconnected, or a case where data reception from the sensor transmitter to the terminal is stopped in a state where the communication connection is established.

In the method, the method may include receiving, by the terminal, biometric information in response to an advertisement transmitted by the sensor transmitter, and the delaying of the display of the communication module turn-off for the one period of time may include delaying the display of the communication module turn-off for the one period of time starting from one advertisement timing after a time point when the communication module is turned off.

In the method, the method may include receiving, by the terminal, biometric information in response to an advertisement transmitted by the sensor transmitter, and the delaying of the display of the communication module turn-off for the one period of time may include delaying the display of the communication module turn-off for the one period of time from a time point when the communication module is turned off.

As described above, according to the embodiments, by determining whether the communication module is turned off while waiting for a predetermined period of time during which the terminal delays the display of the communication module turn-off, the turn-off of the communication module may be accurately determined and a notification may be provided to the user in a timely manner.

### BRIEF DESCRIPTION OF THE DRAWINGS

FIG. 1 is a diagram for schematically explaining a glucose monitoring system according to an embodiment.
FIG. 2 is a diagram for explaining an applicator for attaching a sensor transmitter to a human body according to an embodiment.
FIG. 3 is a diagram for explaining a process of attaching a sensor transmitter to a human body using an applicator according to an embodiment.
FIG. 4 is a configuration diagram of a sensor transmitter according to an embodiment.
FIG. 5 is a configuration diagram of a terminal according to an embodiment.
FIG. 6 is an example diagram in which biometric information is generated in a sensor transmitter or a terminal according to an embodiment.
FIG. 7 an example diagram in which a data packet is generated in a sensor transmitter according to an embodiment.
FIG. 8 is a flowchart for explaining a method of transmitting and receiving biometric information between a sensor transmitter and a terminal according to an embodiment.
FIG. 9 is a diagram for explaining an example of displaying a communication status by a terminal according to an embodiment.
FIG. 10 is a diagram for explaining another example of displaying a communication status by a terminal according to an embodiment.
FIG. 11 is an example diagram of a user interface provided by a terminal to display a communication status according to an embodiment.
FIG. 12 is a flowchart for explaining a method of displaying a communication status by a terminal according to an embodiment.
FIG. 13 is a flowchart for specifically explaining a method of displaying a communication status by a terminal according to an embodiment.
FIG. 14 is a diagram for explaining a method of displaying a communication status when receiving an advertisement message from a sensor transmitter by a terminal according to an embodiment.

### DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

Terms such as first and second are merely identifiers used to distinguish identical or corresponding components, and the identical or corresponding components are not limited by terms such as first and second.

Hereinafter, embodiments according to the present disclosure will be described in detail with reference to the accompanying drawings, and in describing with reference to the accompanying drawings, identical or corresponding components will be assigned the same drawing numbers and overlapping descriptions thereof will be omitted.

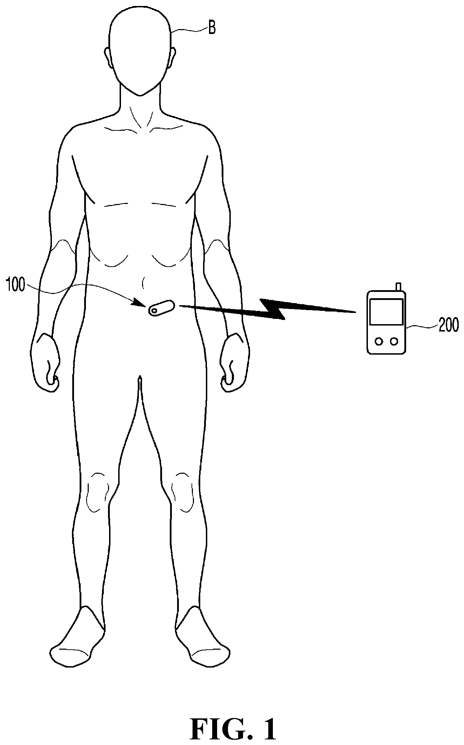

FIG. 1 is a diagram for schematically explaining a glucose monitoring system according to an embodiment.

Referring to FIG. 1, a glucose monitoring system 10 (hereinafter referred to as a "system") according to an embodiment may include a sensor transmitter 100 and a terminal 200.

The sensor transmitter 100 is attached to a human body B, and when the sensor transmitter 100 is attached to the human body B, one end of a sensor of the sensor transmitter 100 is inserted into the skin to periodically extract body fluids from the human body and measure glucose.

The terminal 200 may receive a biosignal including glucose information from the sensor transmitter 100, generate glucose information from the biosignal, and output to the user. The terminal 200 may include, but is not limited to, various devices such as smartphones, mobile phones, tablet PCs, desktops, and laptops, and may have a communication interface capable of communicating with the sensor transmitter 100 and may include a device on which a program or application may be installed.

The sensor transmitter 100 may periodically transmit measured biosignals to the terminal 200 at the request of the terminal 200 or at a set time. For data communication between the sensor transmitter 100 and the terminal 200, the sensor transmitter 100 and the terminal 200 may be connected to each other via a wired connection such as a USB cable or wirelessly using methods such as infrared communication, NFC communication, or Bluetooth.

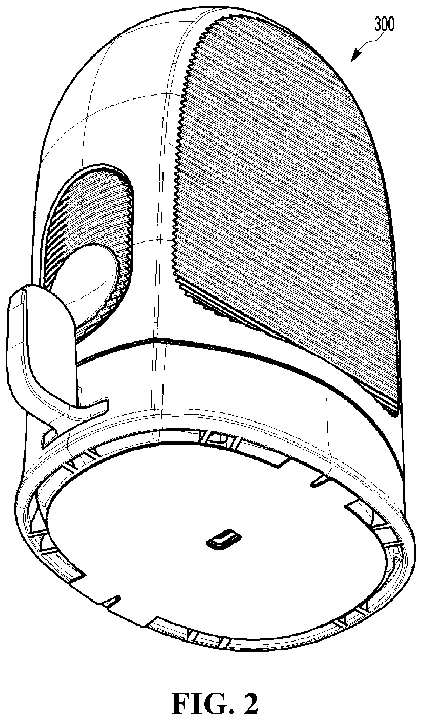

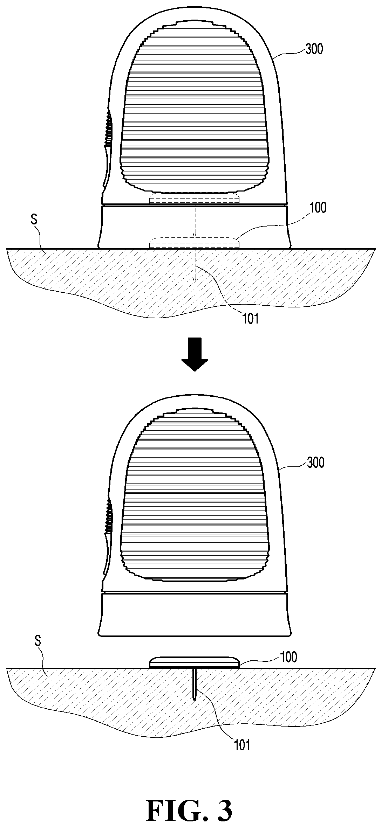

FIG. 2 is a diagram for explaining an applicator for attaching a sensor transmitter to a human body according to an embodiment, and FIG. 3 is a diagram for explaining a process of attaching a sensor transmitter to a human body using an applicator according to an embodiment.

Referring to FIGS. 2 and 3, an applicator 300 according to an embodiment has the sensor transmitter 100 inside and operates to discharge the sensor transmitter 100 to the outside and attach it to a specific body part of the user through manipulation by the user. The applicator 300 is formed in a shape with one side open, and the sensor transmitter 100 is installed in the applicator 300 through the open side of the applicator 300.

When attaching the sensor transmitter 100 to a part of the body using the applicator 300, in order to insert one end of a sensor provided in the sensor transmitter 100 into the skin, the applicator 300 may include a needle (not shown) formed to surround one end of the sensor inside, a first elastic member (not shown) that pushes the needle and one end of the sensor together into the skin, and a second elastic member (not shown) for pulling out only the needle. Through this configuration of the applicator 300, the needle and one end of the sensor may be simultaneously inserted into the skin by decompressing the first elastic member (not shown) disposed in a compressed state inside the applicator 300. When one end of the sensor is inserted into the skin, only the needle is pulled out by decompressing the compressed second elastic member (not shown). The user may safely and easily attach the sensor transmitter 100 to the skin through the applicator 300.

Looking in detail at the process of attaching the applicator 300 to the human body B, with a protective cap (not shown) removed, the open side of the applicator 300 is brought into close contact with the skin S of a specific area of the human body B. When the applicator 300 is operated in this way while the applicator 300 is in close contact with the skin S of the human body B, the sensor transmitter 100 is discharged from the applicator 300 and may be attached to the skin S. Here, one end of the sensor 101 is disposed at the lower part of the sensor transmitter 100, exposed from the sensor transmitter 100, and one end of the sensor 101 may be partially inserted into the skin S through the needle provided in the applicator 300. Therefore, the sensor transmitter 100 may be attached to the skin S with one end of the sensor 101 inserted into the skin S.

Here, an adhesive tape may be provided on the surface of the sensor transmitter 100 in contact with the human body B so that the sensor transmitter 100 may be fixedly attached to the skin S of the human body B. Therefore, when the applicator 300 is separated from the skin S of the human body B, the sensor transmitter 100 may be fixedly attached to the skin S of the human body B by the adhesive tape.

Afterwards, when power is applied to the sensor transmitter 100, the sensor transmitter 100 communicates with the terminal, and the sensor transmitter 100 may transmit biosignals including glucose information to the terminal. The sensor transmitter 100 may generate not only glucose information but also various biometric information, and hereinafter, it will be explained that glucose information is measured as an example of biometric information.

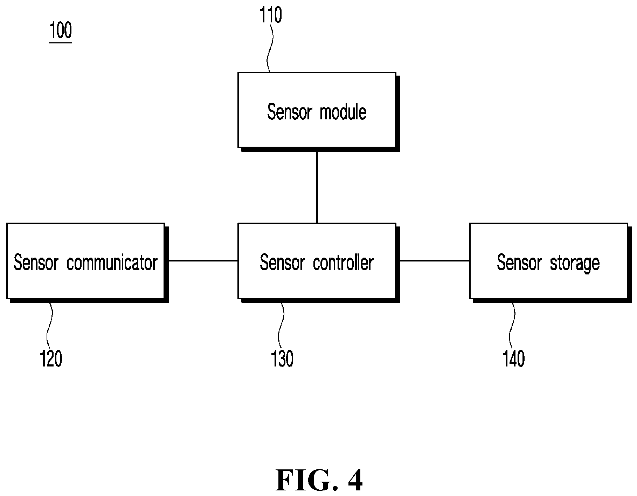

FIG. 4 is a configuration diagram of a sensor transmitter according to an embodiment.

Referring to FIG. 4, the sensor transmitter 100 according to an embodiment may include a sensor module 110, a sensor communicator 120, a sensor controller 130, and a sensor storage 140.

The sensor module 110 may include at least one sensor that is inserted into the human body and senses biomass. At least one sensor may measure biomass and generate biosignals. The biosignal is an analog signal and may include a current value.

The sensor communicator 120 may exchange data or information with the terminal. For example, the sensor communicator 120 may transmit biosignals received from the sensor module 110 or data stored in the sensor storage 140 (for example, biometric information) to the terminal.

The sensor controller 130 may control the overall configuration of the sensor transmitter 100, including the sensor module 110, the sensor storage 140, and the sensor communicator 120. For example, the sensor controller 130 may receive a control signal from the terminal and control the configuration of the sensor transmitter 100 accordingly. In addition, the sensor controller 130 may process biosignals. For example, the sensor controller 130 may convert biosignals into analog or digital form or perform processing to remove noise as needed.

Data or information may be stored in the sensor storage 140. For example, the sensor storage 140 may store data on biomass measured by the sensor module 110, for example, the current value of the biosignal or its digital form data, or data received from the terminal, for example, the command value of the control signal.

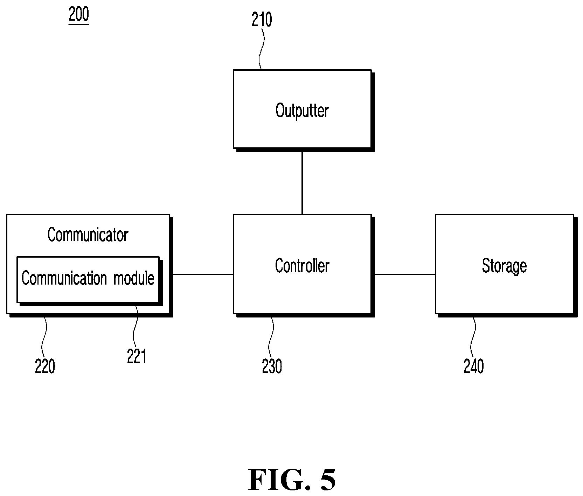

FIG. 5 is a configuration diagram of a terminal according to an embodiment.

Referring to FIG. 5, the terminal 200 according to an embodiment may include an outputter 210, a communicator 220, a controller 230, and a storage 240.

The outputter 210 may output biometric information included in the biosignal, for example, glucose information, so that the user may check it. For example, the outputter 210 may display glucose information as a numerical level (value) or a graph processed from the numerical value. In addition, the outputter 210 may output the communication status between the sensor transmitter 100 and the terminal 200. The communication status may be visually represented differently depending on the type of status. For example, a state in which communication is smooth ( when a communication connection is established and biometric information is received) may be displayed in blue, a state in which the terminal does not receive biometric information for a predetermined period of time (when a communication connection is established but biometric information is not received) may be displayed in red, and a state in which the communication module is turned off or is not connected to the sensor (when a communication connection is not established) may be displayed in gray.

The communicator 220 may communicate with the sensor communicator of the sensor transmitter and exchange data or information. For example, the communicator 220 may receive a biosignal containing information about biomass (i.e., biometric information) measured by the sensor transmitter. Here, the communicator 220 may receive primarily processed biosignals from the sensor transmitter. Preferably, the processed biosignal may include discrete data (discontinuous data) in which a current value, which is an analog signal, is converted into digital data. If the current value is sampled every cycle, digital discrete data may be generated. Alternatively, the communicator 220 may transmit a control signal for controlling the sensor transmitter to the sensor transmitter.

In addition, the communicator 220 may include a communication module 221 therein. The communication module 221 may be a component that performs an independent function together with other components of the communicator 220. The communication module 221 may include a component for executing a specific communication interface. For example, the terminal 200 may communicate with the sensor transmitter 100 in a manner such as USB, infrared, NFC, or Bluetooth, and the communication module 221 may be a functional component specialized for each communication interface. Preferably, the communication module 221 may be a Bluetooth module for supporting Bluetooth-type communication. The communicator 220 basically includes an antenna, a digital signal processor (DSP), and the like, necessary for communication, and the communication module 221 may support Bluetooth-type communication together with these.

Data or information may be stored in the storage 240. For example, data received from the sensor transmitter, for example, biometric information, may be stored in the storage 240. Here, biometric information may include glucose information, and may include digital data representing current values. Alternatively, data input from the user or environment setting data for setting the operating environment of the terminal may be stored in the storage 240.

The controller 230 may include at least one processor that executes a program that displays a communication status in a glucose monitoring system and at least one memory in which the program is stored. The memory and processor included in the controller 230 may be integrated into one chip or may be physically separated.

Memory may be implemented as non-volatile memory devices such as read only memory (ROM), programmable ROM (PROM), erasable programmable ROM (EPROM), electrically erasable programmable ROM (EEPROM), and flash memory, or volatile memory devices such as random access memory (RAM) to store various programs, data, and/or information.

In addition, the controller 230 may generate a glucose value, which is quantified glucose information, from biometric information. To this end, the controller 230 may obtain biometric information in the form of a current value from the sensor transmitter, and preprocess and/or process the current value of the biometric information. The controller 230 may first calculate the sensitivity and generate the glucose value according to this sensitivity.

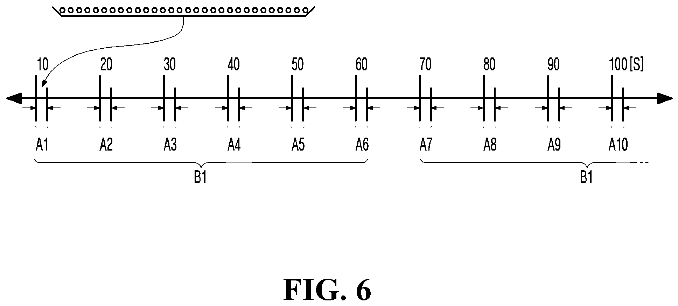

FIG. 6 is a diagram illustrating a first example in which biometric information is generated in a sensor transmitter according to an embodiment.

Referring to FIG. 6, biometric information may be generated in a sensor transmitter according to an embodiment. Specifically, the sensor transmitter may obtain an analog (continuous) biosignal representing a current value at predetermined intervals, and sample the biosignal to generate digital (discontinuous) data representing the current value. The generated data may be processed in the sensor transmitter and biometric information may be generated. Hereinafter, it is described that biometric information is generated by generating digital data from a biosignal in the sensor transmitter and processing it, but is not limited thereto, and part or all of the processing may be performed in the sensor transmitter depending on the embodiment.

For example, the sensor transmitter may obtain a biosignal in an analog form, for example, a current value, measure the biosignal every 10 seconds, and process the measured biosignal to generate a single first data. Specifically, the sensor transmitter may measure (sample) a biosignal 30 times every 10 seconds and generate digital data. The sensor transmitter may remove upper data and lower data from the 30 pieces of data, calculate an average value (A1) of the remaining data, and determine this average value (A1) as the single first data. The first data, which is the data of the average value (A1) calculated in this manner, is generated in 10 second-units, and as illustrated, six average values (A1 to A6), i.e., six pieces of first data, may be generated in one minute.

In addition, the sensor transmitter may process 30 pieces of first data every 300 seconds (5 minutes).

The sensor transmitter may generate an average value (B 1) again using the six pieces of first data (average values (A1 to A6)). In generating the average value (B1), the terminal may remove the upper data and lower data among the six average values (A1 to A6) and generate the average value (B 1) of the remaining data. The second data, which is the data of the average value (B1) calculated in this way, is generated in 1 minute-units, and as illustrated, one average value (B1), i.e., one piece of second data, may be generated in one minute.

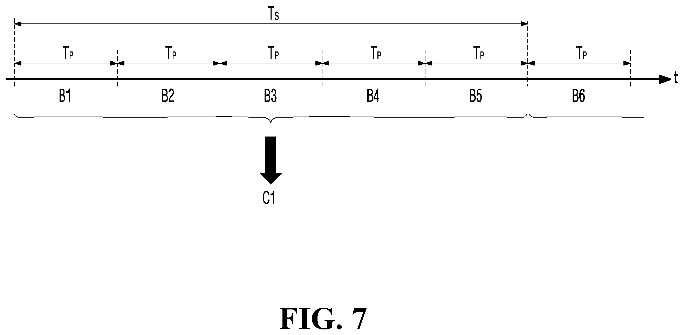

FIG. 7 is a diagram illustrating a second example in which biometric information is generated in a sensor transmitter according to an embodiment.

Referring to FIG. 7, second data may be processed to generate biometric information in a sensor transmitter according to an embodiment. In the above-described example, the sensor transmitter may obtain 5 pieces of second data (B1) in 1 minute-units from 6 pieces of first data in 10 second-units. In addition, the sensor transmitter may generate an average value (C1) using the 5 pieces of second data (average values (B1 to B5)). In generating the average value (C1), the terminal may remove upper data and lower data among the 5 average values (B1 to B5) and generate an average value (C1) of the remaining data. The third data, which is the data of the average value (C1) calculated in this way, is generated in 5-minute units, and as illustrated, one average value (C1), i.e., one piece of third data, may be generated in 5 minutes.

Here, the terminal may sequentially generate second data (B1 to B5) during a biometric information generation period (Tp) and generate one third data (C1) during a glucose value biometric information generation period (Ts). In the glucose value biometric information generation period (Ts), a glucose value is calculated from the third data (C1) through sensitivity, and in the biometric information generation period (Tp), second data (B1 to B5) that serve as the basis for the third data (C1) may be generated. In the example described above, the biometric information generation period (Tp) may correspond to 1 minute, and the glucose value biometric information generation period (Ts) may correspond to 5 minutes.

In this way, the third data generated in 5-minute units may undergo a noise removal process by a filter. The filtered third data may be converted into biometric information including a glucose value by applying sensitivity, and such biometric information may be output to the user.

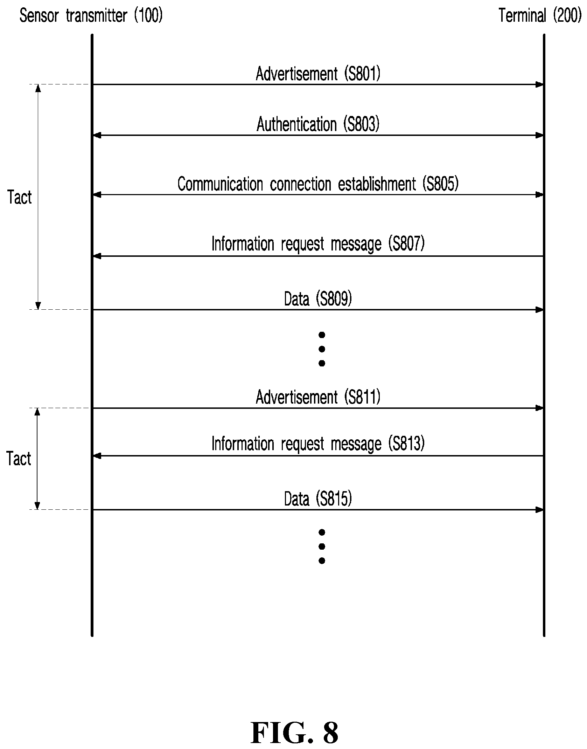

FIG. 8 is a flowchart for explaining a method of transmitting and receiving biometric information between a sensor transmitter and a terminal according to an embodiment.

Referring to FIG. 8, the sensor transmitter 100 and the terminal 200 according to an embodiment may connect communication in order to transmit and receive data including biometric information. Data may be transmitted and received after the communication connection. The sensor transmitter 100 and the terminal 200 may be connected to each other through wired or wireless communication, and may connect communication in a manner such as USB communication, infrared communication, or Bluetooth communication.

Specifically, the sensor transmitter 100 and the terminal 200 may transmit and receive differently depending on whether communication is disconnected and a new communication is connected (when connecting initial communication) or only data is transmitted and received after initial communication is connected. First, in a state of communication disconnection, when the sensor transmitter 100 and the terminal 200 establish a new communication connection, the sensor transmitter 100 may advertise to the terminal 200 (step S801). The sensor transmitter 100 may generate a constant signal for advertisement and transmit this advertisement signal to the terminal. Alternatively, the sensor transmitter 100 may perform advertisement by periodically transmitting an advertisement message to the terminal 200. The process of sending this advertisement signal or advertisement message may be called advertisement. The terminal 200 may receive the advertisement signal or advertisement message and perform authentication with the sensor transmitter 100 (step S803). For example, the sensor transmitter 100 and the terminal 200 may authenticate that they are valid devices through a hash value. Then, the sensor transmitter 100 and the terminal 200 may establish a communication connection (step S805). The communication connection is a state in which data may be transmitted and received immediately without going through a separate initial communication process such as authentication, and the sensor transmitter 100 and the terminal 200 may transmit and receive data in response to an advertisement at any time while the communication connection is being established. The terminal 200 may transmit an information request signal or an information request message to the sensor transmitter 100 to obtain data including biometric information (for example, 30 pieces of first data) (step S807). The sensor transmitter 100 that has received the information request signal or information request message may transmit data including biometric information (for example, 30 pieces of first data) to the terminal 200 in response thereto (step S809).

Once the initial communication is connected, data may be transmitted and received in the communication connection establishment state without a separate authentication process. The sensor transmitter 100 may repeatedly advertise to the terminal 200 in the following operation section (step S811). Repeated advertisement may be performed by periodically or aperiodically transmitting an advertisement signal or an advertisement message to the terminal 200.

The terminal 200 may send an information request message requesting data transmission in response to the advertisement to the sensor transmitter 100 (step S813). The sensor transmitter 100 may transmit data in response to the information request signal or information request message (step S815).

Here, the terminal 200 receives data from the sensor transmitter 100 from time to time, but the terminal 200 may receive data in response to the advertisement message only when the sensor transmitter 100 advertises. The sensor transmitter 100 may send this advertisement signal or advertisement message to the terminal 200 periodically or aperiodically, and the terminal 200 may request and receive data accordingly. **In** addition, the sensor transmitter 100 may not continuously send the advertisement signal or advertisement message, but may transmit it only in active mode, i.e., while awake. Accordingly, data transmission and reception between the sensor transmitter 100 and the terminal 200 may be performed only during this active mode period (Tact). The sensor transmitter 100 may stand by without sending any data during the inactive mode, i.e., the non-active mode period.

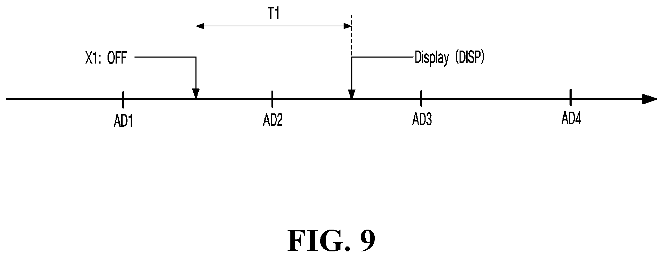

FIG. 9 is a diagram for explaining an example of displaying a communication status by a terminal according to an embodiment.

Referring to FIG. 9, an example of displaying a communication status by a terminal according to an embodiment may be illustrated. In this example, when the communication module is turned off, the terminal may delay the display that the communication module is turned off for one period of time and wait to display the turn-off of the communication module.

Specifically, the communication module may be turned off at time point X1 during communication between the sensor transmitter and the terminal. The turn-off indicates a state in which the communication module stops functioning, and may be intentionally set by a user or caused by an internal error. The controller of the terminal may detect whether the communication module is turned off. In addition, the terminal may not immediately display the communication module turn-off and may wait for one period of time (T1). When the one period of time (T1) has elapsed, the terminal may finally display the communication module turn-off (DISP). However, in order for the terminal to display the communication module turn-off, it may be required that a communication failure persists for the one period of time (T1). If the terminal receives data from the sensor transmitter during the one period of time (T1) under any circumstances, the original communication status (for example, communication module turn-on) may be displayed without displaying the communication module turn-off.

Here, the communication failure may mean a state in which data cannot be received due to a communication module (e.g., a Bluetooth communication module) being turned off or due to any failure (e.g., when the distance between the sensor transmitter and the terminal increases) even when the communication module is turned on. The former example corresponds to a case in which the communication connection between the sensor transmitter and the terminal is cut off, and the latter example corresponds to a case in which the terminal stops receiving data from the sensor transmitter while the communication connection is established. The terminal may determine whether there is a communication failure based on the operating status of the communication module and whether data is received. Therefore, the terminal may determine whether such a communication failure persists during the one period of time (T1), and if the communication failure persists, may display the communication module turn-off after the one period of time (T1).

In addition, the terminal may receive biometric information from the sensor transmitter in response to an advertisement sent by the sensor transmitter. If a communication failure occurs, even if the sensor transmitter transmits a signal or message to the terminal for advertisement, the terminal may not receive the signal or message. Alternatively, even if the signal or message reaches the terminal, the terminal may not be able to transmit the information request message or receive data including biometric information in response to the information request message. At the time point when the sensor transmitter advertises, i.e., at the respective advertisement timing (AD1, AD2, AD3, AD4), the terminal may not be able to receive data from the sensor transmitter as described above.

If no communication failure occurs, the advertisement timing (AD1, AD2, AD3, AD4) may, in principle, include the following characteristics. The advertisement timings (AD1, AD2, AD3, AD4) may be periodic or aperiodic. When the advertisement timings (AD1, AD2, AD3, AD4) are periodic, the advertisement timings (AD1, AD2, AD3, AD4) may be formed at intervals of 1 minute. At the advertisement timings (AD1, AD2, AD3, AD4), i.e., at 1-minute intervals, the sensor transmitter may advertise to the terminal and send a signal or message for this advertisement to the terminal. In addition to the advertisement, the sensor transmitter may transmit data including biometric information to the terminal at the advertisement timings (AD1, AD2, AD3, AD4). Strictly speaking, when the sensor transmitter collects data from the sensor for a predetermined period of time, the collected data may be transmitted to the terminal at any one of the advertisement timings (AD1, AD2, AD3, AD4). As in the example described above, the sensor transmitter may collect and process data for 300 seconds (5 minutes) and transmit it to the terminal. The sensor transmitter may continuously collect first data in 10 second-units, generate second data in 60 second (1 minute)-units from the first data, and generate third data in 300 second (5 minute)-units from the second data. When the 300-second (5-minute) amount of data (i.e. the third data) is completed at any one of the advertisement timings (AD1, AD2, AD3, AD4), the sensor transmitter may send the 300-second (5 minute) amount of data to the terminal at once. For example, if the sensor transmitter has already completed the 300-second (5-minute) amount of data at the advertisement timing (AD1), the sensor transmitter may perform only advertising at the advertisement timings (AD2, AD3, AD4) without transmitting data. If the sensor transmitter should have completed the 300-second (5-minute) amount of data at the advertisement timing (AD1) but fails to do so, the sensor transmitter may complete the 300-second (5-minute) amount of data by the next advertisement timing (AD2, AD3, or AD4) and transmit it to the terminal. The advertisement timings (AD1, AD2, AD3, AD4) are repeated, and other advertisement timings may be formed at 1-minute intervals thereafter.

Here, advertisement timings (AD1, AD2, AD3, AD4) may be named as the first to fourth advertisement timings, respectively. Here, the one period of time (T1) may proceed immediately from the time point when the communication module is turned off regardless of the advertisement timings (AD1, AD2, AD3, AD4), and if the communication failure persists during the one period of time (T1), the communication module turn-off may be displayed after the one period of time (T1) has elapsed.

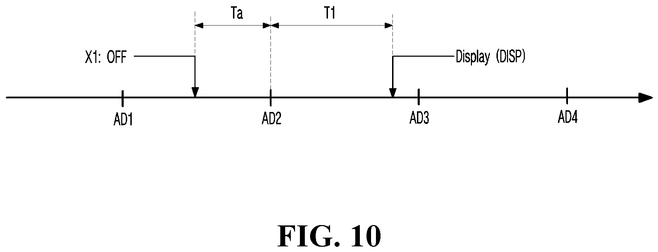

FIG. 10 is a diagram for explaining another example of displaying a communication status by a terminal according to an embodiment.

Referring to FIG. 10, another example of displaying a communication status by a terminal according to an embodiment may be illustrated. In the above-described example, one period of time (T1) may proceed from time point X1 where the communication module is turned off. On the other hand, in another example, one period of time (T1) may proceed from the first advertisement timing that arrives first after time point X1, and the terminal may only display the communication module turn-off when the one period of time (T1) has elapsed from the advertisement timing. Accordingly, a period during which the terminal delays the display of the communication module turn-off may be longer than the one period of time (T1).

For example, if the communication module is turned off at time point X1, the terminal may delay the display of the communication module turn-off until the second advertisement timing (AD2) that arrives first after time point X1, i.e., for a period a (Ta), and may wait for the display. Additionally, the terminal may delay the display of the communication module turn-off for one period of time (T1) starting from the second advertisement timing (AD2) and wait for the display. Therefore, if the communication failure persists for the period a (Ta) and the one period of time (T1), the terminal may indicate the communication module turn-off only after the period a (Ta) and the one period of time (T1).

The one period of time (T1), which is the period during which the terminal delays the display as described above, may be preset when the terminal is shipped or may be set by a user input. The user may set the start time, the end time, and the conditions for starting and ending the one period of time (T1).

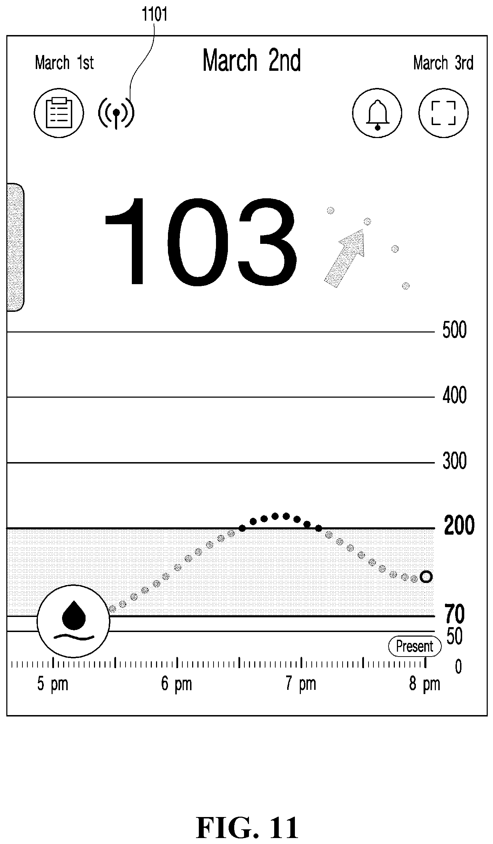

FIG. 11 is an example diagram of a user interface provided by a terminal to display a communication status according to an embodiment.

Referring to FIG. 11, an example of a user interface implemented to display a communication status in an application according to an embodiment may be illustrated. A controller of the terminal may execute an application, and an outputter may visually display a user interface implemented by the application. The outputter may obtain biometric information from the controller and display it on the user interface.

The user interface may include an icon 1101 indicating a communication status (e.g., a Bluetooth communication connection). The icon 1101 may visually display the communication status differently depending on the type. For example, the icon 1101 may indicate a state in which communication is smooth (for example, a case where a communication connection is established and biometric information is received) as blue, a state in which the terminal does not receive biometric information for a predetermined period of time (for example, a case where a communication connection is established but biometric information is not received) as red, and a state in which the communication module is turned off or is not connected to the sensor for communication (for example, a case where no communication connection is established) as gray. Accordingly, when the communication module is turned off, the icon 1101 may change to gray.

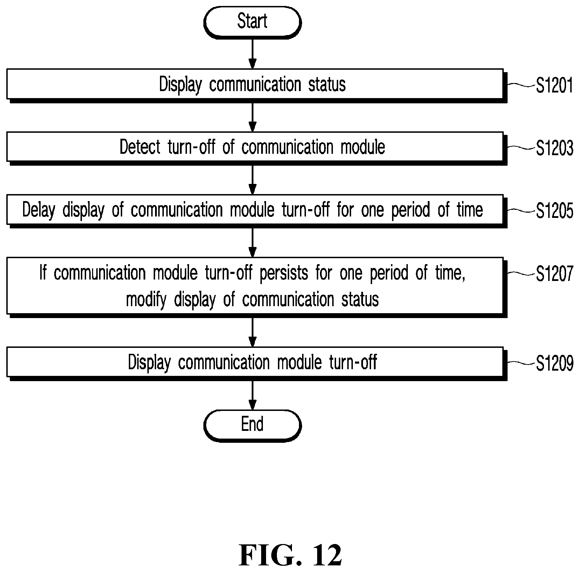

FIG. 12 is a flowchart for explaining a method of displaying a communication status by a terminal according to an embodiment.

Referring to FIG. 12, a method of displaying a communication status of a terminal according to an embodiment may be illustrated. When the communication module is turned off, the status of the communication module may be detected, a grace period for displaying the communication module turn-off may be provided, and after determining whether the communication failure persists, the communication module turn-off may be displayed.

Specifically, the controller of the terminal may execute an application, and the outputter may display the communication status through the user interface (step S1201). The outputter may visualize the communication status, such as the turn-off and turn-on of the communication module, the case where data is not received while the communication module is turned on, and the case where the communication module is not connected to the sensor transmitter while the communication module is turned on, in various ways.

When the communication module is turned off, the controller of the terminal may detect the communication module turn-off (step S1203).

In addition, the controller of the terminal may delay the display indicating that the communication module is turned off for one period of time (step S1205). The controller of the terminal may make the terminal wait while the display is delayed. Then, the controller of the terminal may determine whether the communication failure persists. If the communication failure persists for the one period of time, the controller of the terminal may delay the display of the communication module turn-off and make the terminal wait while the communication failure persists.

If the one period of time has elapsed while the communication failure persists, the controller of the terminal may control the outputter to modify the display of the communication status (step S1207). For example, the controller of the terminal may modify the icon of the user interface showing the communication status to indicate the communication module turn-off. In addition, the outputter of the terminal may display the modified icon (step S1209).

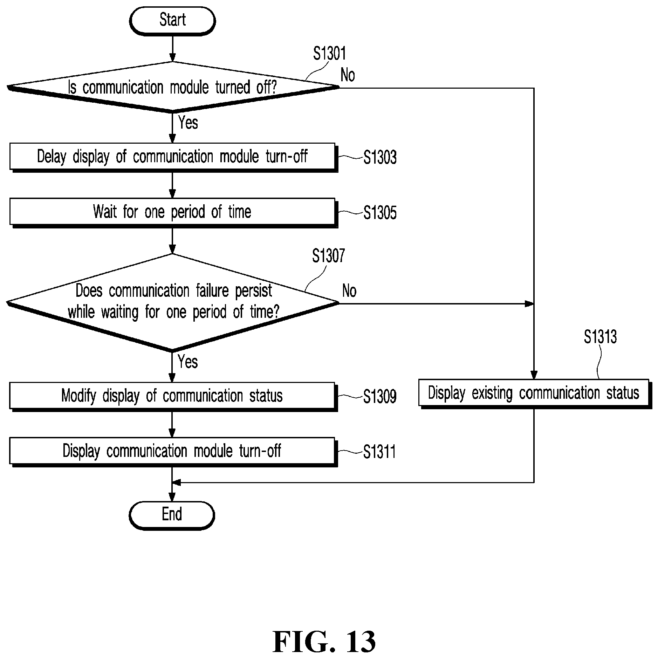

FIG. 13 is a flowchart for specifically explaining a method of displaying a communication status by a terminal according to an embodiment.

Referring to FIG. 13, a method of displaying a communication status by a terminal according to an embodiment may be illustrated in detail. The terminal may delay and wait for a grace period to display the communication module turn-off. During the grace period, the terminal may determine whether to display the communication module turn-off, and these determinations may be performed during the grace period. In the present drawing, the description will be centered on the determinations performed by the terminal during one period of time.

The controller of the terminal may determine whether the communication module is turned off (step S1301). If it is determined that the communication module is not turned off, the currently maintained communication status may be displayed (NO of step S1301 and S1313).

If it is determined that the communication module is turned off, the terminal may perform determination on whether to display the communication module turn-off for a predetermined period of time. To this end, first, the controller of the terminal may delay the display of the communication module turn-off (step S1303). The controller of the terminal may make the terminal wait for the display of the communication module turn-off (step S1305). Then, the controller of the terminal may perform determination on whether to display the communication module turn-off (step S1307). If it is determined that the communication failure does not persist, the terminal may display the existing communication status that is currently maintained (NO of step S1307 and S1313).

If it is determined that the communication failure persists, the controller of the terminal may control the outputter to modify the display of the communication status (YES of step S1307 and step S1309). Now, the communication status indicates the communication module turn-off, and the outputter of the terminal may output the display indicating the communication module turn-off (step S1311).

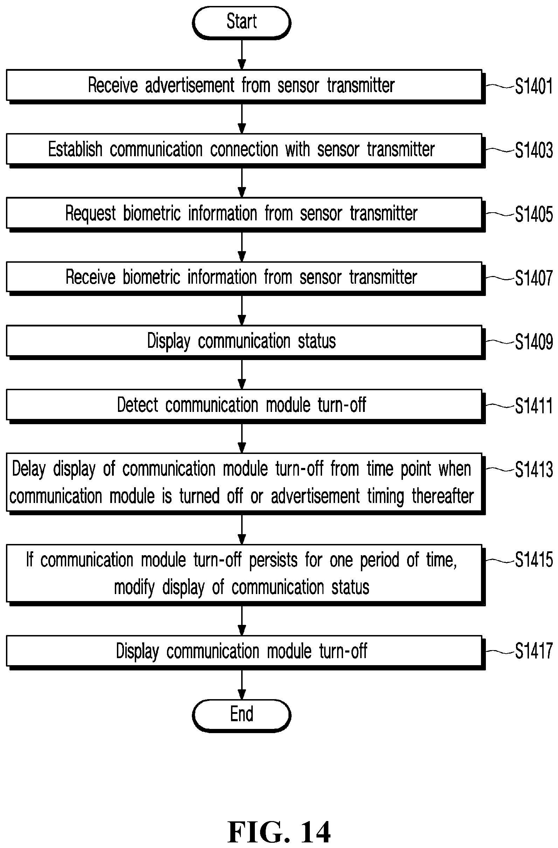

FIG. 14 is a diagram for explaining a method of displaying a communication status when receiving an advertisement message from a sensor transmitter by a terminal according to an embodiment.

Referring to FIG. 14, a method of displaying a communication status in a situation where a terminal receives biometric information in response to an advertisement from a sensor transmitter according to an embodiment may be illustrated.

The terminal may periodically receive an advertisement from the sensor transmitter (step S1401). The terminal may request and establish a communication connection to the sensor transmitter through a signal or message for the advertisement, and may request biometric information based thereon (steps S1403 and S1405). The terminal may receive biometric information from the sensor transmitter in response to the request (step S1407). The terminal may display the communication status to indicate that data transmission and reception are smooth (step S1409).

The communication module may be turned off, and the terminal may detect that the communication module is turned off (step S1411). The terminal may delay the display of the communication module turn-off for one period of time starting from the time point when the communication module is turned off or the advertisement timing thereafter (step S1413). The terminal may determine whether the communication failure persists while waiting for the one period of time.

If a communication failure, i.e., a state in which the communication module is turned off or data is not received even when the communication module is turned on, persists and the one period of time has elapsed, the terminal may modify the display of the communication status to indicate the communication module turn-off (step S1415). Then, the terminal may output the display of the modified communication status (i.e., the display of the communication module turn-off) (step S1417).

The aspects of the subject matter described herein may be described in the context of computer-executable instructions, such as program modules, that are executed on a computer. Generally, program modules include routines, programs, objects, components, data structures, etc., which perform specific tasks or specific abstract data types.

Alternatively or additionally, the functionality described herein may be performed, at least in part, by one or more hardware logic components. By way of example, and not limitation, exemplary types of hardware logic components that may be used include field-programmable gate array (FPGA), program-specific integrated circuit (ASIC), application-specific standard product (ASSP), system-on-a-chip system (SOC), complex programmable logic device (CPLD), and the like.

In addition, the disclosed embodiments may be implemented in the form of a non-transitory recording medium that stores programs and/or instructions executable by a computer. Instructions may be stored in the form of program code, and when executed by a processor, may create program modules to perform operations of the disclosed embodiments. The non-transitory recording medium may be implemented as a non-transitory computer-readable recording medium

Computer-readable recording media include all types of recording media storing instructions that may be decoded by a computer. For example, there may be read only memory (ROM), random access memory (RAM), magnetic tape, magnetic disk, flash memory, optical data storage, and the like.

Although embodiments of the present disclosure have been described above, those skilled in the art will be able to make various modifications and changes to the present disclosure by adding, changing or deleting components, without departing from the present disclosure as set forth in the appended claims, which are included within the scope of rights of the present disclosure.

## Claims

1. A method of displaying a communication status in a glucose monitoring system, the method comprising:
displaying (S1201) a communication status of a communication module in a terminal configured to transmit and receive data with a sensor transmitter;
when the communication module is turned off, delaying (S1205) a display of a communication module turn-off for one period of time by the terminal; and
if the turn-off of the communication module persists for the one period of time, displaying (S1209) the communication module turn-off.

2. The method according to claim 1, wherein the communication status indicates a Bluetooth communication connection between the terminal and the sensor transmitter.

3. The method according to claim 1, wherein the one period of time is preset or input by a user.

4. The method according to claim 1, further comprising:
performing (S1307) determination on whether to display the communication module turn-off,
wherein the displaying (S1209) of the communication module turn-off comprises displaying the communication module turn-off based on a result of the determination.

5. The method according to claim 4, wherein the performing (S1307) of the determination on whether to display the communication module turn-off comprises determining whether to display the communication module turn-off based on whether a communication failure persists in the terminal for the one period of time, and
the displaying (S1209) of the communication module turn-off comprises, if the communication failure persists for the one period of time, displaying the communication module turn-off.

6. The method according to claim 1, wherein the communication failure comprises a case where a communication connection between the terminal and the sensor transmitter is disconnected, or a case where data reception from the sensor transmitter to the terminal is stopped in a state where the communication connection is established.

7. The method according to claim 1, further comprising:
Receiving (S1407), by the terminal, biometric information in response to an advertisement transmitted by the sensor transmitter, and
wherein the delaying (S1205) of the display of the communication module turn-off for the one period of time comprises delaying the display of the communication module turn-off for the one period of time starting from one advertisement timing after a time point when the communication module is turned off.

8. The method according to claim 1, further comprising:
Receiving (S1407), by the terminal, biometric information in response to an advertisement transmitted by the sensor transmitter, and
wherein the delaying (S1205) of the display of the communication module turn-off for the one period of time comprises delaying the display of the communication module turn-off for the one period of time from a time point when the communication module is turned off.
